(19) Europäisches Patentamt
European Patent Office
Office européen des brevets

(11) **EP 3 975 964 B1**

(12) **EUROPEAN PATENT SPECIFICATION**

(45) Date of publication and mention
of the grant of the patent:
**16.07.2025 Bulletin 2025/29**

(21) Application number: **20751845.7**

(22) Date of filing: **03.06.2020**

(51) International Patent Classification (IPC):
*A61F 13/513* (2006.01)   *A61F 13/514* (2006.01)
*A61F 13/537* (2006.01)

(52) Cooperative Patent Classification (CPC):
**A61F 13/51394; A61F 13/51496; A61F 13/537**

(86) International application number:
**PCT/US2020/070110**

(87) International publication number:
**WO 2020/247972 (10.12.2020 Gazette 2020/50)**

(54) **DISPOSABLE ABSORBENT ARTICLES**

SAUGFÄHIGE EINWEGARTIKEL

ARTICLES ABSORBANTS JETABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2019 US 201962856407 P**

(43) Date of publication of application:
**06.04.2022 Bulletin 2022/14**

(73) Proprietor: **The Procter & Gamble Company**
**Cincinnati, OH 45202 (US)**

(72) Inventors:
• **CARLIN, Edward, Paul**
  **Cincinnati, Ohio 45202 (US)**
• **PATEL, Sonam, Jayanti**
  **Cincinnati, Ohio 45202 (US)**
• **HARDIE, Stephen, Lebouf**
  **Cincinnati, Ohio 45202 (US)**
• **GLASSMEYER, Ronda, Lynn**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**US-A1- 2008 249 494      US-A1- 2014 343 525**
**US-A1- 2016 051 419      US-A1- 2019 117 475**

**Description**

FIELD OF THE INVENTION

The present invention pertains to disposable absorbent articles suitable for absorbing and containing body exudates.

BACKGROUND OF THE INVENTION

A variety of disposable absorbent articles have been relied on by consumers to handle or manage body exudates. These consumers may include babies, toddlers, children, teenagers, adults, and elderly persons. Thus, it is clear that the types of fluids or body exudates managed by such articles may vary as well to include urine, feces, menses, and other discharges. Typically, in the case of adults, the articles take the form of sanitary napkins, adult incontinence pads, and adult incontinence diapers or undergarments. One of the primary drivers of the desirability of these products to wearers is to give them assurance that when they experience incontinence, the occurrence of such will go unnoticed by others and even more ideally by the wearers.

Some manufacturers utilize visual signals to communicate to the wearer of the extent of the absorbent layers of the article. These visual signals can be particularly useful, especially when the absorbent article in question is relatively thin. Visual signals can help alleviate a wearer's anxiety regarding the threat of leakage from the article.

However, providing visual signals to communicate the extent of the absorbent layers can be problematic. The absorbent layers are generally disposed inboard of a periphery of the disposable absorbent article. This ensures that there is minimal leakage from the absorbent layers outside of the periphery of the article. That said, due to web tracking issues, printing on the absorbent layers generally means that the print signal can be disposed several centimeters inboard of the periphery both longitudinally and laterally. And, while printing on the topsheet can allow for a longer and possibly wider signal, printing on the topsheet can create a risk of ink bleeding onto a wearer's skin.

US2008/249494A1 is concerned with hygiene articles, such as feminine pads, for collecting bodily fluid. The articles include a be substantially white central fluid acquisition zone and two visually discernible lateral zones situated on opposite sides of the central zone. The lateral zones are formed by a nonwoven material including a colored pigment.

US2014/343525A1 relates to an absorbent personal care article having a longitudinal centerline and a transverse centerline and including a pair of opposed wings extending along the longitudinal sides of the article. The article further has two protective strips attached to the sides of the article and extending laterally outboard of the article and the edges of the wearer's undergarment to provide additional protection against leakage.

US2019/117475A1 discloses an absorbent hygiene article for placement in underwear. The topsheet is of a translucent material and is provided with a colored pattern defining at least one background region and at least one colored region having a first inherent color. The topsheet-facing side of the backsheet has a second color; and the background region located in an inner area has a higher L *-value than the background region located in the edge area.

US2016/051419A1 relates to absorbent articles, such as tampons and sanitary napkins, comprising one or more graphical elements. The graphical elements highlight the functional area of the absorbent article.

Based on the foregoing, there is a need to provide an absorbent article with a more dimensionally robust visual signal - one that communicates a more accurate account of the extent of the absorbent layers.

SUMMARY OF THE INVENTION

Described herein are disposable absorbent articles that assurance to users regarding their absorbent capacity. The absorbent articles described herein comprise a visual signal having a length which is a large percentage of the overall length of the absorbent article.

Disposable absorbent articles constructed in accordance with the present disclosure have a longitudinal centerline and a lateral centerline generally perpendicular to the longitudinal centerline, first and second side edges and first and second end edges disposed at a first end and a second end of the absorbent article, respectively. The absorbent article further comprises a topsheet; a backsheet; an absorbent system disposed between the topsheet and the backsheet; and a visual signal visible from a wearer-facing surface. And, the visual signal of the absorbent articles of the present disclosure have a length wherein a difference between a length of the disposable absorbent article and the length of the visual signal is less than 35 mm. The absorbent articles exhibit a performance factor of between 2 and 13. The performance factor is calculated by dividing the difference between the article length and visual signal length by the measured caliper

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction

The disposable absorbent articles, particularly incontinence pads or pants, of the present invention can provide flexibility to allow for an improved and comfortable fit which is less susceptible to bunching during use. In particular, it is envisioned that the articles of the present disclosure exhibit heightened structural resiliency from the proposed configuration and orientation of the layers contained therein. For the purposes of this disclosure, reference to an incontinence pad, disposable absorbent article, or absorbent article will be used. However, the present invention may be applied to a plurality of absorbent articles including, but not limited to, sanitary napkins, pantiliners, menstrual pads, diapers, training pants, adult incontinence pants, etc.

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

"Longitudinal" means a direction running substantially perpendicular from a waist edge to a longitudinally opposing waist edge of an absorbent article when the article is in a flat out, uncontracted state, or from a waist edge to the bottom of the crotch, i.e. the fold line, in a bi-folded article. Directions within 30 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 30 degrees of the lateral direction are considered to be "lateral."

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The disposable absorbent articles, particularly incontinence pads or pants, of the present disclosure can provide flexibility to allow for an improved and comfortable fit which is less susceptible to bunching during use. In particular, it is envisioned that the articles of the present disclosure exhibit heightened structural resiliency from the proposed configuration and orientation of the layers contained therein while also allowing for conformance of the article.

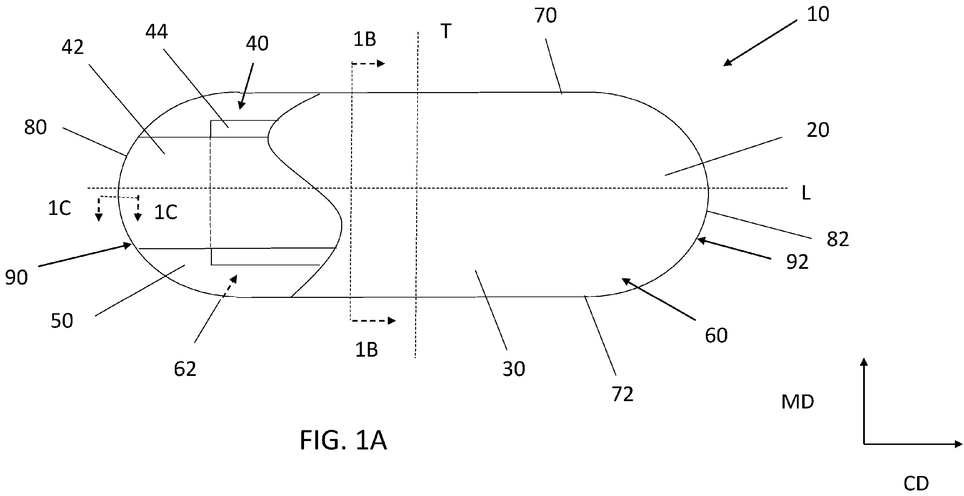

FIG. 1A shows an absorbent article 10 according to the present disclosure. The absorbent article 10 comprises a longitudinal centerline L and a transverse centerline T. The longitudinal centerline L generally extends parallel to the longest dimension of the absorbent article 10. The transverse centerline T extends generally perpendicular to the longitudinal centerline L and lies in the same plane as the absorbent article 10 in a flattened state on a flat surface. The transverse centerline T generally bisects the length of the absorbent article 10 where the length is parallel to the longitudinal centerline L, and the longitudinal centerline L generally bisects the width of the absorbent article 10 where the width is parallel to the transverse centerline T. Additionally, as shown, the MD direction (machine direction) may be generally parallel to the longitudinal centerline L of the absorbent article 10, and the CD direction (cross-machine direction) may be generally parallel to the transverse centerline T.

The absorbent article 10 further comprises a chassis 20 which comprises a topsheet 30, a backsheet 50, and an absorbent system 40 comprising at least one absorbent core sandwiched between the topsheet 30 and the backsheet 50. As shown, the topsheet 30 forms a portion of a wearer-facing surface 60 (the surface of the absorbent article which is disposed most proximal to the wearer during use). And, the backsheet 50 forms a portion of a garment-facing surface 62 (the surface of the absorbent article which is most proximal to the garment of the wearer during use).

The absorbent system 40 may comprise a secondary topsheet 42 and at least one absorbent layer 44. As shown, the secondary topsheet 42 can be disposed between the topsheet 30 and the absorbent layer 44. Additionally, at least one layer of the absorbent system 40 may extend longitudinally to a first end 90 and/or a second end 92 of the absorbent article 10. As shown, the secondary topsheet 42 can extend to the first end 90 or the second end 92. Or, the secondary topsheet 42 and/or additional layers of the absorbent system may extend the full length of the absorbent article 10 (first end 90 to the second end 92 along the longitudinal centerline L).

Additionally, the absorbent article 10 further comprises a first side edge 70 and a second side edge 72. Each of the first side edge 70 and the second side edge 72 extend generally parallel to the longitudinal centerline L. It is worth noting however that the first and second side edges are not required to be straight lines. Instead, the first and the second side edges can be curvilinear or any other suitable shape.

A first end edge 80 joins the first side edge 70 and the second side edge 72 in the first end 90. A second end edge 82 joins the first side edge 70 and the second side edge 72 in the second end 92. Cumulatively, the first side edge 70, the second side edge 72, the first end edge 80 and the second end edge 82 form an outer periphery of the absorbent article 10.

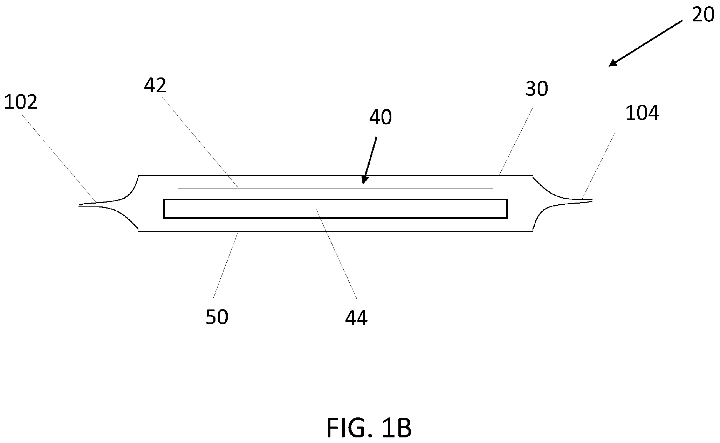

Referring now to FIGS. 1A and 1B, the outer periphery of the absorbent article 10 generally comprises crimp seals 102 and 104 which join the topsheet 30 and the backsheet 50. The crimp seals 102 and 104 correspond to the first side edge 70 and the second side edge 72. Crimp sealing of the topsheet and backsheet is known in the art. As shown, none of the layers of the absorbent system 40 may be disposed within the crimp seals 102 and 104. Some suitable examples of crimp sealing are further described in WO99/25295 and WO97/07764. Crimp sealing typically involves the application of heat and pressure to bond materials together. For example, in the case of the crimp seals 102 and 104, the topsheet and the backsheet may be bonded together via crimp sealing.

Referring now to FIGS. 1A-1C, the outer periphery of the absorbent article 10 also comprises a first end seal 110 which corresponds to the first end edge 80. Additionally, the absorbent article 10 comprises a second end seal which corresponds to the second end edge 82. In contrast to the crimp seals 102 and 104, the first edge seal 110 may comprise the secondary topsheet 42 in addition to the topsheet 30 and the backsheet 50. Similarly, in conjunction with the first edge seal 110 or independently thereof, the second edge seal may comprise the secondary topsheet 42 along with the topsheet 30 and the backsheet 50. It is worth noting that the absorbent layer 44 in conjunction with or independent of the secondary topsheet 42 may be disposed in at least one of the first or second edge seal.

In general, since the layers of the absorbent system 40 are absorbent, manufacturers are hesitant for these materials to be disposed in any crimp / edge seals of the article. Because the layers are absorbent, these layer typically are more efficient at moving liquid insults away from the topsheet toward the absorbent layer 44. Additionally, these layers can sometimes be engineered to spread a liquid insult longitudinally and transversely along the pad so that the more of the absorbent layer 44 is effectively utilized. This spreading of liquid insult could occur even in a crimp / edge seal if not carefully designed. Unfortunately, this spreading of the liquid insult could lead to fluid leakage from the article.

However, the inventors have found that when the absorbent article is changed at regular intervals, in accordance with usage instructions, one or more of the layers of the absorbent system 40 can be included in the first edge seal and/or second edge seal, while minimizing the risk of fluid leakage from this inclusion. In order to minimize the risk of leakage, one or more of the layers of the absorbent system 10 may be phobically treated adjacent the crimp / edge seals. Treatments for the hydrophobic treatment of nonwoven webs are known.

Additionally, absorbent materials typically comprise materials which do not respond positively to crimp sealing. Namely, crimp sealing of absorbent materials typically does not provide sufficient bond strength to maintain product integrity through use. So, where the first edge seal 110 and/or the second edge seal comprise the secondary topsheet 42 and/or the absorbent layer 44, additional provisions may need to be implemented to ensure that sufficient bond strength is achieved. The inventors have found that a combination of adhesive and crimp sealing can provide sufficient bond strength to the absorbent article.

Referring now to FIGS. 1A-2, the absorbent article 10 may further comprise a visual signal 230 that comprises one or more elements. For example, as shown, the visual signal 230 may comprise hearts, moons, stars, clouds, rainbows, flowers, the like or combinations thereof. Any suitable design can be utilized. The visual signal 230 is visible from the wearer-facing surface 60 such that a user can see the visual signal during application of the absorbent article. The visual signal 230 has a visual signal length 260 which can be determined in accordance with the article length and length of visual signal method as disclosed herein. Similarly, the absorbent article 10 can have a length 270 which can be determined in accordance with the article length and length of visual signal method as disclosed herein.

The visual signal 230 can be provided on a layer of the absorbent system 40, on the underside of the topsheet, and/or a portion of the backsheet. For example, the visual signal 230 may be printed on the secondary topsheet 42 or may be printed on the absorbent layer 44. However, the visual signal 230, as mentioned previously, should be visible from the wearer-facing surface 60. As such, the layers which comprise the visual signal should be carefully selected to ensure that the printing is visible by the user from the wearer-facing surface 60. Still, the visual signal may be printed on multiple layers of the absorbent system 40. As an example, a portion of the visual signal may be printed on the secondary topsheet 42 and another portion of the visual signal may be printed on the absorbent layer 44. If the portion of the visual signal on the absorbent layer 44, is disposed outboard of the bounds of the secondary topsheet 42, then that portion of the visual signal may be visible from the wearer-facing surface 60.

As noted previously, the visual signal can provide the user with reassurance that the absorbent article has sufficient capacity to absorb and retain fluid insults. This is particularly relevant where the overall absorbent product is thin, e.g. less than about 9 mm thick. Because at least one layer of the absorbent system 40 extends to the first end 90 and/or the second end 92, the visual signal length 260 can extend along a larger percentage of the absorbent article length 270. The visual signal length 260 is discussed in additional detail hereafter.

In general, thicker pads are perceived by users as having sufficient capacity to absorb liquid insults. Thinner pads, e.g. less than about 9 mm thick, typically have to employ other mechanisms to convince users that the thinner pad has sufficient absorbent capacity. This is typically done via the provision of a visual signal. The inventors have found that the provision of a visual signal can provide the user with confidence that the absorbent article will absorb liquid insults without leaking. For example, the inventors have found that visual signal patterns which resemble walls or boundaries near the extremes of the absorbent article can provide reassurance to the user. In one particular example, arcuate designs near the ends or sides of the absorbent article can be effective at communicating boundaries which can communicate the notion of containment of the liquid insult, e.g. keeping the liquid insult away from the edges of the absorbent article. The provision of this type of visual signal can provide the user with confidence that the fluid will not go beyond the edges of the absorbent system. The inventors have also found that visual signals which are more extensive (cover a higher percentage length of the length of the article) can be more effective at communicating the assurance to the user of the sufficient absorbent capacity of the absorbent article. These longer visual signals can include ovals, arcuate shapes, etc. and may comprise a deep color to help communicate to the user the sufficient absorbent capacity of the absorbent system of the absorbent article. The relationship between visual signal length and caliper is discussed in additional detail hereafter.

It is worth noting that - not according to the claimed invention - visual signals which extend the full length of the pad may be utilized. In such constructions, the layer upon which the visual signal is printed may be printed by a material supplier or by the absorbent article manufacturer. The material supplier or absorbent article manufacturer may print the material of the absorbent system with a visual signal. The absorbent article manufacturer can then cut the printed material to length during processing. However, in order for the visual signal to achieve the resemblance of a boundary, registration of the visual signal may be required. Registration could minimize the likelihood that a portion of the visual signal, e.g. a boundary, is cut off during the manufacturing process.

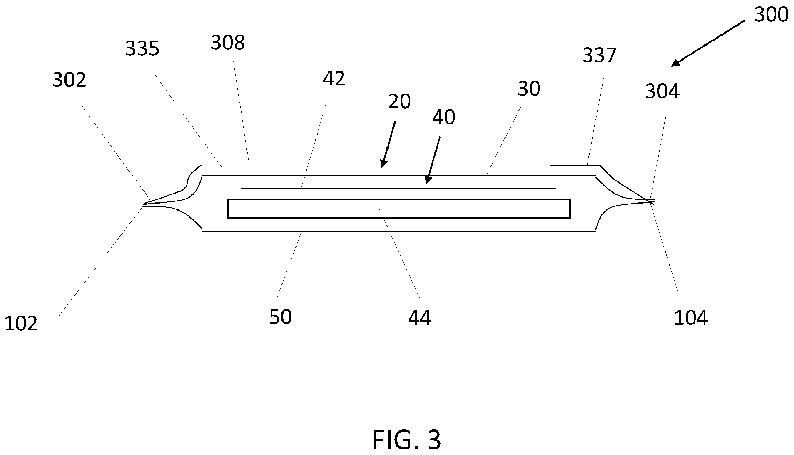

Referring now to FIG. 3, an absorbent article 300 may comprise the chassis 20 as described herein and may further comprise a pair of non-elasticized barrier cuffs 335 and 337. "Nonelasticated" as used herein means the absence of elastic elements beyond the natural elasticity inherently found in the material itself. Thus, the barrier cuffs do not include any rubber thread,

The first and second barrier cuffs 335, 337 are provided at least in a central portion of the absorbent article 300, however, it will be appreciated that the first and second barrier cuffs may extend longitudinally from the first end edge 90 to the second end edge 92 of the absorbent article. The first and second cuffs 335, 337 may be attached directly to the wearer-facing surface 60 of the absorbent article, for example, the topsheet 30, or they may be attached at any other point on the absorbent article, for example, to the backsheet 50. The first and second barrier cuffs extend towards the longitudinal centerline of the absorbent article.

Post manufacture, the first and second barrier cuffs typically lie flush with the topsheet, extending upwards only upon disturbance to the pad, for example, by folding for packing or during use by a consumer. The barrier cuffs serve to provide some extra protection to the absorbent article, particularly upon initial insult of the article with, for example, a sudden gush of urine. In such circumstances, it may take some time for the exudates to penetrate the absorbent article and reach the absorbent core. The first and second cuffs help limit sideways leakage from the top surface of the absorbent article.

The first and second cuffs have a proximal end 302 and 304 attached between the longitudinal centerline and a longitudinal edge of the chassis. Preferably, the proximal end of the respective barrier cuffs is coterminous with the side crimp edge seals 102 and 104 of the chassis, however, it will be appreciated that the proximal end of the barrier cuffs may also be located inboard of the longitudinal edge of the chassis. A distal end 308 of each of the first and second barrier cuffs extends towards the longitudinal centerline of the absorbent article. For the avoidance of doubt, at least in a central section of the absorbent article, the distal end of each of the first and second cuffs is not attached to the topsheet or chassis. Thus, the distal end of the first and second cuffs in at least the central section of the chassis is free to lift up from topsheet, thus impeding lateral flow of exudates from the surface of the absorbent article.

Preferably, the width of the first and second barrier cuffs, i.e., that distance measured between the proximal end and the distal end in a direction substantially orthogonal to the longitudinal centerline is at least 3mm, 4mm, 5mm, 6mm, 7mm or 8mm. Without being bound by theory, it is thought that the cuffs need a minimum width to be able to separate from the topsheet and to "stand-up" relative to the topsheet. Where the width of the cuffs varies along the length of the respective cuffs, the width is measured at the lateral center point of the absorbent article. This typically coincides with the minimum width of cuff in the central section of the absorbent article.

Preferably, the distal end of one or both of the first and second barrier cuffs extends over a portion of the absorbent core. Without being bound by theory, it is thought that the differential thickness and stiffness in the core area relative to the chassis increases the ease with which the cuffs may separate from the topsheet and "stand-up" relative to the topsheet. Thus, preferably, the first and second barrier cuffs extend over the absorbent core by at least 0.5 mm, 1 mm, 1.5 mm, 2 mm, 2.5 mm, 3 mm or 3.5 mm. Furthermore, to ensure that a sufficient surface area of the topsheet above the core is exposed to the user's body, the first and second barrier cuffs may extend over a total of between 1% and 70% of the width of the core $W_c$, as measured at the widest part of the core. At least 30% of the core should remain exposed to enable exudates to be absorbed.

Preferably, the extent to which the first and second barrier cuffs extend over the absorbent core does not exceed 40%, 35%, 30%, 25%, 20%, 15% or 10% of the overall width $W_{aa}$ of the absorbent article, as measured at its widest point. For example, for a pad having a maximum width of 100mm, each cuff would be between 3mm and 40mm wide as measured along the widest point $W_{AA}$ of the absorbent article. There is a balance between providing cuffs that are wide enough to hold a sufficient amount of liquid, without being so wide that they become too heavy to lift off the topsheet and/or so wide that they cover the core making it harder for liquids to be absorbed.

The first and second barrier cuffs may be formed of one or more layers of material. Providing more than one layer effectively increases the overall stiffness and strength of the cuff, thus enabling it to more easily separate from the topsheet and therefore stand-up relative to the topsheet, thus providing better protection while in use. The multiple layers also provide better protection against liquid passing through the cuffs and more strength when fluids enter under the cuff.

Where the first and/or second cuffs include multiple layers, the cuffs may be formed of multiple strips of material attached together, either at the attachment strip, or at a distal end. Alternatively, the cuffs may comprise one or more folds formed of a single piece of material to form cuffs with multiple layers. Where the cuff is folded, one, all or select layers of the folded cuff may be attached directly to the chassis. Where the first and second cuffs comprise a single piece of material folded at a distal end of the cuff to form a dual layer cuff, where only one end of the material is attached to the chassis and where the other end of the material is attached to the cuff itself. Where two layers of material make up the cuff and are folded back on one another, then a portion of the combined material is folded back at the distal end.

Providing a fold in the material biases the cuff to more naturally separate itself from the topsheet during use. Without being bound by theory, it is thought that the fold introduces some natural resilient tension into the cuff, such that the cuff has a natural tendency to return to an unfolded state, which causes the cuffs to "stand-up" relative to the topsheet.

Using multiple layers increases the collective stiffness of the cuff relative to using a single layer of material with a higher basis weight. Thus, it is possible to use thinner material, for example, the topsheet material, and to achieve the benefits of a stiffer material that results in separation of the cuffs from the topsheet and enables the cuffs to stand up relative to the topsheet.

The cuffs may be formed of the same material as the topsheet. Using the same material as the topsheet reduces manufacturing costs, for example by enabling bulk order of material and by reducing complexity at the manufacturing line by reducing the number of unwinds (and corresponding space) needed for introducing extra materials. Where the cuffs are formed of the same material as the topsheet, they may be treated to make them phobic.

The first and second cuffs may be made from hydrophilic or hydrophic materials, topsheet or backsheet, Spunbond meltblown spunbond (SMS) or other nonwovens with melt blown layers. The cuffs may also be made of synthetic or natural fibers such as polyethylene, polypropylene, polyester materials, polyamides (e.g., nylon), cotton, silk or pulp which has been spunbonded, carded, melt blown, or a combination of similar known methods. The first and second cuffs may be made from the same material as one of the topsheet or the backsheet, thus reducing raw material costs. Preferably, the cuffs are formed of SMS. Additional disclosure regarding the non-elasticized cuffs is provided in U.S. Patent Application Serial No. 62/772750 filed on November 29, 2018, entitled "Absorbent Articles."

In contrast, additional absorbent articles are contemplated which utilize elasticized barrier cuffs. Unlike the non-elasticized cuffs described heretofore, the elasticized cuffs comprise elastic members which can contract and expand. Otherwise the elasticized barrier cuffs may be configured similarly to the non-elasticized barrier cuffs exception the provision of elastic members attached to the cuff material. Additional disclosure regarding absorbent articles with elasticized barrier cuffs can be found in U.S. Patent Application Publication No. 2017/0049634A1.

Additionally, arrays of products are contemplated which include absorbent articles comprising non-elasticized barrier cuffs and elasticized barrier cuffs. For example, absorbent articles can be sized depending upon expected loading. Some users may require an absorbent article with a small amount of absorbency (light absorbency), some may require an intermediate amount of absorbency (medium absorbency), and some may require a high amount of absorbency (high absorbency). It is worth noting that in general, the distinction between light and medium absorbency is via length of the absorbent article. Light absorbency absorbent articles are generally less than 250 mm in length while medium absorbency articles are generally greater than 250 mm in length. High absorbency articles generally are in a different product form than their light and medium counterparts. For example, light and medium absorbency articles are generally in pad form while high absorbency articles are typically in pant form.

The difference between light, medium, and high absorbency articles may be highlighted in packaging via a plurality of droplet icons. As an example, a light absorbent article will have 2 droplets highlighted, while a medium absorbency article will have more than two droplets highlighted, e.g. 3 or 4, while a high absorbency article will have 4 or more droplets highlighted. It is worth noting that droplet icons are not required for this indication, droplet icons are merely an example of an indicator which can be used.

There are several ways to accommodate this variation in needs. One way may be that manufacturer's increase the length of the pads for the various sizes. For example, a user requiring light absorbency may utilize an article having a first length while users requiring medium absorbency may utilize an article having a second length. The second length may be greater than the first length. Another way, to accommodate the variation may be to increase the basis weight of the absorbent layer. For example, an absorbent article for light absorbency may have a first basis weight while another absorbent article for medium absorbency has a second basis weight. The second basis weight may be greater than the first basis weight. Still another way to accommodate the variation in absorbency needs is to provide the user with a combination of the foregoing. For example, variations in article length and absorbent layer basis weights may be provided in an array.

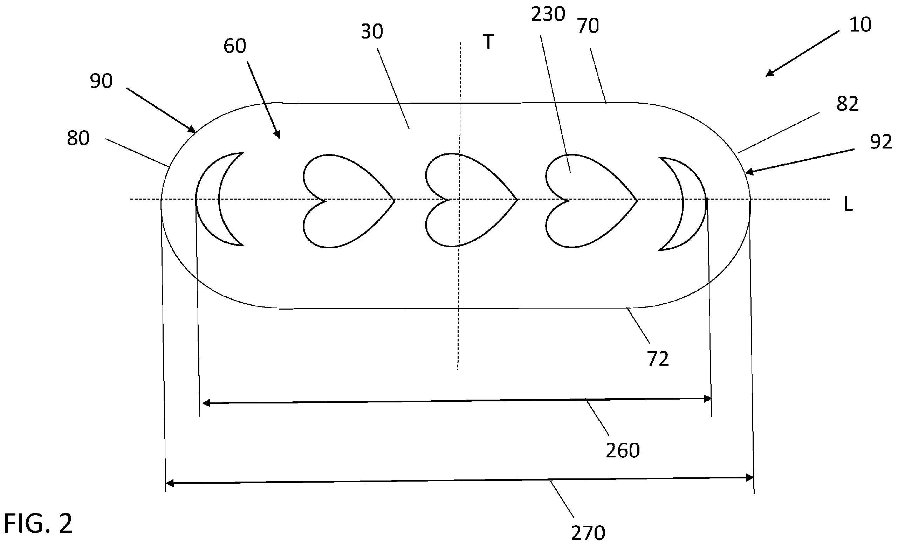

Referring back to FIG. 2, arrays of absorbent articles in accordance with the present disclosure, may comprise a plurality of absorbent articles that can accommodate the variation in needs discussed previously. A first plurality of absorbent articles can accommodate light absorbency needs. The first plurality of absorbent articles can have a visual signal length 260 determined in accordance with the visual signal length method of between about 140 mm to about 230 mm, more preferably from about 150 mm to about 220 mm, or most preferably from about 160 mm to about 210 mm, specifically including all values within these ranges and any ranges created thereby. The first plurality of absorbent articles can have an overall article length 270 of between 160 mm to about 260 mm, more preferably from about 170 mm to about 250 mm, or most preferably from about 180 mm to about 240 mm, specifically reciting all values within these ranges and any ranges created thereby.

A second plurality of absorbent articles can accommodate medium absorbency needs. The second plurality of absorbent articles can have visual signal length 260 as determined in accordance with the visual signal length method of between about 220 mm to about 400 mm, more preferably from about 235 mm to about 390 mm, or most preferably from about 240 mm to about 370 mm, specifically reciting all values within these ranges and any ranges created thereby. The second plurality of absorbent articles can have an overall article length 270 of between 230 mm to about 430 mm, more preferably from about 240 mm to about 420 mm, or most preferably from about 250 mm to about 410 mm, specifically reciting all values within these ranges and any ranges created thereby.

It should be understood there may be additional pluralities of absorbent articles which can accommodate light absorbency needs. Similarly, there may be additional pluralities of absorbent articles which can accommodate medium absorbency needs. Each of these additional pluralities of articles may have articles lengths as described herein.

The first plurality of absorbent articles and any other plurality of articles which are meant to accommodate light absorbency needs, can comprise the non-elasticized cuffs as disclosed herein. Additionally, or independently thereof, these absorbent articles may comprise a visual signal length 260 which is at least about 84 percent of the absorbent article length 370, more preferably at least about 86 percent, or most preferably about 88 percent, specifically reciting all values within these ranges and any ranges created thereby.

Where a number of absorbent article sizes are offered with regard to light absorbency, the visual signal length 260 can be at least about 81 percent of the absorbent article length 270 where the absorbent article length is between about 140 mm and about 175 mm. Where the absorbent article length 270 is between about 175 mm to about 190 mm, the visual signal length 260 can be at least about 84 percent of the absorbent article length 270. Where the absorbent article length 270 is between about 190 mm to about 230 mm, the visual signal length 260 can be at least about 85 percent of the absorbent article length 270. For these articles the visual signal length can be between about 130 mm to about 220 mm or more preferably from about 130 mm to about 210 mm, specifically reciting all values within these ranges and any ranges created thereby.

The second plurality of absorbent articles and any other articles which are meant to accommodate medium absorbency needs, can comprise non-elasticized cuffs. Or, because of the increased capacity of the medium absorbency articles, these absorbent articles may comprise elasticized cuffs. Additionally, or independently thereof, these absorbent articles may comprise a visual signal length 260 which is at least about 80 percent of the absorbent article length 370, more preferably about 85 percent, most preferably at least about 90 percent, specifically reciting all values within these ranges and any ranges created thereby.

Where a number of absorbent article sizes are offered with regard to the medium absorbency, the visual signal length 260 can be at least about 75 percent of the absorbent article length 270 where the absorbent article length is between about 230 mm to about 270 mm. Where the absorbent article length 270 is between about 270 mm to about 328 mm, the visual signal length 260 can be at least about 77 percent of the absorbent article length 270. Where the absorbent article length 270 is between about 328 mm to about 390 mm, the visual signal length 260 can be at least about 80 percent of the absorbent article length 270.

Some exemplary article and visual signal lengths are provided below in Table 1 with regard to light absorbency articles.

Table 1

| Size | Light 1 | Light 2 | Light 3 |
| --- | --- | --- | --- |
| Article length (mm) | 190 | 221 | 240 |
| Visual Signal length (mm) | 160 | 191 | 210 |

(continued)

| Size | Light 1 | Light 2 | Light 3 |
|---|---|---|---|
| Percentage | 84 | 86 | 88 |

It is worth noting that the difference between the article length (mm) and the visual signal length (mm) can be indicative of the relative distance of the visual signal from an end edge of the absorbent article. For example, for Light 1, the difference between the absorbent article length and the visual signal length is 30 mm. So, the visual signal may be positioned about 15 mm from each end edge of the absorbent article. Of course, the visual signal may be positioned asymmetrically regarding the transverse axis. In such cases, the visual signal may be within about 25 mm of one end of the absorbent article while being within 5 mm of the other end of the absorbent article.

Some exemplary article and visual signal lengths are provided below in Table 2 with regard to medium absorbency articles. Additionally, for these articles, theoretical stack up calipers are provided. Theoretical stack up calipers are simply the cumulative calipers based upon target thickness of the components of the absorbent article.

Table 2

| Size | Medium 1 | Medium 2 | Medium 3 | Medium 4 | Medium 5 |
|---|---|---|---|---|---|
| Article length (mm) | 270 | 317 | 348 | 368 | 400 |
| Visual Signal length (mm) | 240 | 287 | 318 | 338 | 370 |
| percentage | 89 | 91 | 91 | 92 | 93 |
| Delta (article length - visual signal length) (mm) | 30 | 30 | 30 | 30 | 30 |
| Stack up caliper (mm) | 5.4 | 5.8 | 6.1 | 6.8 | 6.7 |
| Measured caliper (mm) | 5.1 | | | | 5.7 |
| PF (stack up) | 6 | 5 | 5 | 4 | 4 |
| PF (measured) | 6 | | | | 5 |

Data regarding visual signals of conventional absorbent articles is provided below in Table 3.

Table 3

| Size | Medium 1 | Medium 2 | Medium 3 | Medium 4 | Medium 5 |
|---|---|---|---|---|---|
| Article length (mm) | 270 | 317 | 348 | 368 | 400 |
| Visual Signal length (mm) | 185 | 229 | 255 | 278 | 306 |
| Percentage | 69 | 72 | 73 | 76 | 77 |
| Delta (article length - visual signal length) (mm) | 85 | 88 | 93 | 90 | 94 |
| Stack up caliper (mm) | 4.7 | 4.8 | 5 | 5 | 5.1 |
| PF (stack up) | 18 | 18 | 19 | 18 | 18.4 |

As previously mentioned, thinner articles tend to require more convincing - so to speak - regarding consumer reassurance. Where the absorbent articles have a caliper of less than about 9 mm, the visual signal and the caliper can be related by a performance factor. The performance factor is the difference between the article length and visual signal length (mm) divided by the measured caliper (mm). For the sake of clarity, the difference is divided by the caliper.

As mentioned previously and not according to the claimed invention, utilization of preprinted materials can offer a visual signal which is the full length of the absorbent article. With this in mind, the performance factor (PF) for the absorbent article of the present disclosure can be greater than or equal to 2 and less than or equal to 13. The performance factor can be between about 2 to about 13, from about 2 to about 10, or from about 3 to about 8, specifically reciting all values within these ranges and any ranges created thereby.

Referring back now to FIG. 1A, as mentioned previously, absorbent articles of the present disclosure comprise the chassis 20 which comprises the topsheet 30, the backsheet 50, and the absorbent system 40 comprising at least one absorbent core sandwiched between the topsheet 30 and the backsheet 50. A discussion of each of these items is provided hereafter.

Arrays of products may comprise a plurality of first packages and a plurality of second packages. The plurality of first packages may comprise first absorbent articles, and the plurality of second packages may comprise second absorbent articles. Each of the first absorbent articles are indicated for lower absorbency than each of the second absorbent articles. For example, the plurality of first packages may have 2 droplets indicated while the plurality of second packages may have 3 droplets highlighted. Further with this example, the first absorbent articles may comprise light absorbency articles, and the second absorbent articles may comprise medium absorbency articles.

Topsheet

The topsheet 30 of the chassis 20 forms a portion of the wearer-facing surface 60. The topsheet 30 may be joined to the absorbent system 40 and to the backsheet 50 by attachment methods (not shown) such as those well known in the art. Suitable attachment methods are described with respect to joining the backsheet 50 to the absorbent system 40. The topsheet 30 and the backsheet 50 may be joined directly to each other in the incontinence pad periphery and may be indirectly joined together by directly joining them to the absorbent system 40. This indirect or direct joining may be accomplished by attachment methods which are well known in the art.

The absorbent article may comprise any known or otherwise effective topsheet, such as one which is compliant, soft feeling, and non-irritating to the wearer's skin. Suitable topsheet materials include a liquid pervious material that is oriented towards and contacts the body of the wearer permitting bodily discharges to rapidly penetrate through it without allowing fluid to flow back through the topsheet to the skin of the wearer. The topsheet, while being capable of allowing rapid transfer of fluid through it, also provides for the transfer or migration of the lotion composition onto an external or internal portion of a wearer's skin. A suitable topsheet can be made of various materials such as woven and nonwoven materials; apertured film materials including apertured formed thermoplastic films, apertured plastic films, and fiber-entangled apertured films; hydroformed thermoplastic films; porous foams; reticulated foams; reticulated thermoplastic films; thermoplastic scrims; or combinations thereof. Some suitable examples of films that can be utilized as topsheets are described in U.S. Patent Nos. 3,929,135; 4,324,246; 4,342,314; 4,463,045; 5,006,394; 4,609,518; and 4,629,643.

Nonlimiting examples of woven and nonwoven materials suitable for use as the topsheet include fibrous materials made from natural fibers, modified natural fibers, synthetic fibers, or combinations thereof. Some suitable examples are described in U.S. Patent Nos. 4,950,264, 4,988,344; 4,988,345; 3,978,185; 7,785,690; 7,838,099; 5,792,404; and 5,665,452.

In some forms, the topsheet may comprise tufts as described in U.S. patent nos. 8,728,049; 7,553,532; 7,172,801; 8,440,286; 7,648,752; and 7,410,683. The primary topsheet may have a pattern of discrete hair-like fibrils as described in U.S. patent no. 7,655,176 or 7,402,723. Additional examples of suitable topsheet includes those described in U.S. Patent Nos. 8,614,365; 8,704,036; 6,025,535 and in U.S. Patent Application Publication Nos 13743M.

Another suitable topsheet or a topsheet combined with a secondary topsheet may be formed from a three-dimensional substrate as detailed in a U.S. Patent Application Publication No. 2017/0258647 A1.

The topsheet may have one or more layers, as described in U.S. Patent Application Publication Nos. 2016/0167334 A1; 2016/0166443 A1; 2017/0258651 A1. The topsheet may be apertured as disclosed in U.S. Patent No. 5,628,097, to Benson et al., issued on May 13, 1997.

Backsheet

The backsheet 50 of the chassis 20 may form a portion of the garment-facing surface 62 of the absorbent article 10. The backsheet 50 may be joined to the absorbent system 40 by attachment methods (not shown) such as those well known in the art. For example, the backsheet 50 may be secured to the absorbent system 40 by a uniform continuous layer of adhesive, a patterned layer of adhesive, or an array of separate lines, spirals, or spots of adhesive. Alternatively, the attachment methods may comprise using heat bonds, pressure bonds, ultrasonic bonds, dynamic mechanical bonds, or any other suitable attachment methods or combinations of these attachment methods as are known in the art. Forms of the present disclosure are also contemplated wherein the absorbent system 40 is not joined to the backsheet 50, the topsheet 30, or both.

The backsheet 50 may be impervious, or substantially impervious, to liquids (e.g., urine) and may be manufactured from a thin plastic film, although other flexible liquid impervious materials may also be used. As used herein, the term "flexible" refers to materials which are compliant and will readily conform to the general shape and contours of the human body. The backsheet 50 may prevent, or at least inhibit, the exudates absorbed and contained in the absorbent system 40 from wetting articles of clothing which contact the absorbent article 10 such as undergarments. However, in some instances, the backsheet 50 may permit vapors to escape from the absorbent system 40 (i.e., is breathable) while in other instances the backsheet 50 may not permit vapors to escape (i.e., non-breathable). Thus, the backsheet 50 may comprise a polymeric film such as thermoplastic films of polyethylene or polypropylene. A suitable material for the backsheet 50 is a thermoplastic film having a thickness of from about 0.012 mm (0.5 mil) to about 0.051 mm (2.0 mils), for example. Any suitable backsheet known in the art may be utilized with the present invention.

Some suitable examples of backsheets are described in US Patent No. 5,885,265; 4,342,314; and 4,463,045. Suitable single layer breathable backsheets for use herein include those described for example in GB A 2184 389, GB A 2184 390, GB A 2184 391, U.S. Pat. No. 4,591,523, U.S. Pat. No. 3 989 867, U.S. Pat. No. 3,156,242; WO 97/24097 and U.S. Patent Nos. 6,623,464; 6,664,439 and 6,436,508.

The backsheet may have two layers: a first layer comprising a gas permeable aperture formed film layer and a second layer comprising a breathable microporous film layer as described in US Patent No. 6,462,251. Suitable dual or multi-layer breathable backsheets for use herein include those exemplified in U.S. Pat. No. 3,881,489, U.S. Pat. No. 4,341,216, U.S. Pat. No. 4,713,068, U.S. Pat. No. 4,818,600, EP 203 821, EP 710 471, EP 710 472, and EP 793 952.

Absorbent System

As mentioned previously, the absorbent system 40 may comprise a plurality of functional layers, e.g. a secondary topsheet and a storage layer. The secondary topsheet 42 may be separate and apart from the absorbent system. Additionally, the secondary topsheet may be disposed beneath the topsheet 30 and on a body-facing surface of the absorbent system 40. The secondary topsheet 42 can server a multitude of functions. First, the secondary topsheet 42 can quickly drain the topsheet from any liquid insults that may be imparted to the topsheet. Second, the secondary topsheet 42 can distribute liquid insults so that the storage layer is utilized more effectively. Namely, the liquid insult may be distributed to a larger area than the expected liquid insult region of the absorbent article.

To accommodate the above functionality, the secondary topsheet may comprise a nonwoven material which comprises a variety of fiber types. For example, the secondary topsheet may comprise absorbing fibers, resilient fibers and stiffening fibers. The absorbing fibers may comprise cellulose or viscose, as an example. The resilient fibers and stiffening fibers may be synthetic. The resilient fibers can help the secondary topsheet retain it void volume by resisting collapsing forces. The stiffening fibers may be bicomponent fibers which can be fused to one another to create a matrix of fiber interconnections. These fiber interconnections can provide the secondary topsheet with stiffening properties.

The secondary topsheet may have a basis weight from about 40 gsm to about 100 gsm, from about 45 gsm to about 75 gsm, or from about 50 gsm to about 60 gsm, specifically including all values within these ranges and any ranges created thereby.

Some exemplary secondary topsheets are described in U.S. Patent Application Publication Nos. 2015/0351976 A1; 2014/0343523 A1; 2018/0098893 A1; and U.S. Patent Application Serial No. 62/826178.

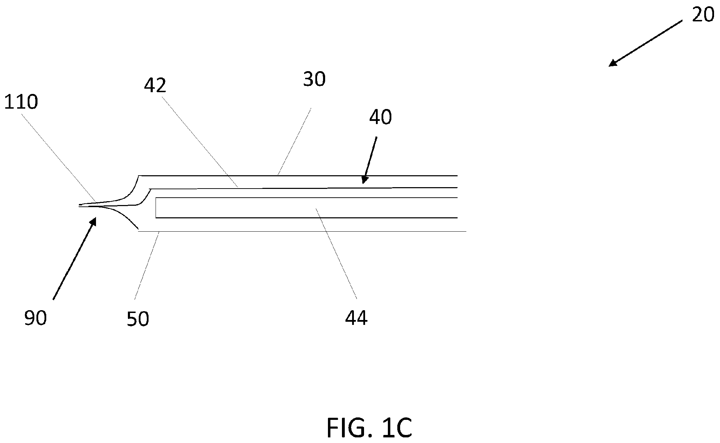

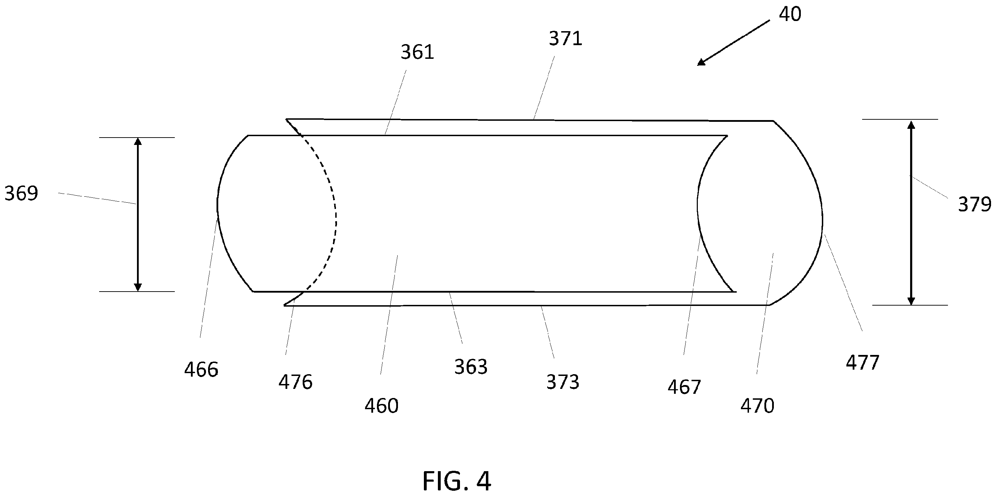

Referring now to FIGS. 1 and 4, the absorbent system 40 either in conjunction with or independently thereof, may comprise a first absorbent core 460 and/or a second absorbent core 470. While the first absorbent core 460 and the second absorbent core 470 are shown as rectangular with rounded ends, any suitable shape may be utilized. Some examples include offset hourglass (one end is wider than an opposite end and a narrowed mid-section between the ends), bicycle seat shape (one end and central portion are narrower than second end), etc. Side edges 70 and 72 may follow the general contour of the first absorbent core 460 and/or the second absorbent core 470. So where, the first absorbent core 460 and/or the second absorbent core 470 are an hourglass shape, the side edges 70, 72 may be arranged in an hourglass shape as well. However, the side edges 70 and 72 may be generally straight or slightly curved such that they do not follow the contour of the first absorbent core 460 and/or the second absorbent core 470. Additional details are discussed hereafter. The absorbent article 10 may be symmetric about the longitudinal centerline L or asymmetric about the longitudinal centerline L. Similarly, the absorbent article 10 may be symmetric about the transverse centerline T or asymmetric about the transverse centerline T.

As shown, the first absorbent core 60 may be positioned in absorbent article more proximal to a wearer-facing surface than the second absorbent core 70. However, forms are contemplated where the second absorbent core 70 is positioned in the absorbent article more proximal to a wearer-facing surface than the first absorbent core 60.

The first absorbent core 460 may comprise side edges 361 and 363 and a pair of end edges 466 and 467 which join the side edges 361 and 363 adjacent the first end 90 and adjacent the second end 92 of absorbent article 10, respectively. Similarly, the second absorbent core 470 may comprise side edges 371 and 373 and a pair of end edges 476 and 477 which join the side edges 371 and 373 adj acent the first end 90 and the second end 92 of the absorbent article 10, respectively. As shown, the first absorbent core 460 has a first width 369 and the second absorbent core 470 comprises a second width 379. As shown, the first width 369 may be greater than the second width 379.

The first absorbent core 460 can have a width 369 ranging from about 39 mm to about 62 mm. The second absorbent core 470 can have a width 379 which ranges from about 50 mm to about 80 mm, from about 58 mm to about 78 mm, specifically reciting all values within these ranges and any ranges created thereby.

Additionally, as shown the end edges 466 and 467 of the first absorbent core 460 may be substantially flat. End edges 476 and 477 of the second absorbent core 470 may be similarly configured. Moreover, as shown, end edge 466 of the first absorbent core 460 may be coterminous with end edge 476 of the second absorbent core 470. Or, the first absorbent core 460 and the second absorbent core 470 may be positioned within the absorbent article 10 such that the first absorbent core 460 is offset from the second absorbent core 470. Such configuration is explained in additional detail hereafter.

Where the first absorbent core 460 and the second absorbent core 470 are offset from one another, the amount of the second absorbent core 470 outboard of the first absorbent core 460 can be constant along the length of the first absorbent core 460. In such forms, it is believed that a minimum portion of the second absorbent core 470 outboard of the first absorbent core 460 is greater than 5 mm. It is believed that this minimum distance can be beneficial in allowing sufficient conformance of the absorbent article. However, forms are contemplated where the portion of the second absorbent core 470 outboard of the first absorbent core 460 may be variable depending on location. Examples regarding this aspect of the absorbent article of the present disclosure are provided hereafter.

Referring now to FIGS. 1, 4, and 5, a cross section of an exemplary absorbent system 40 taken along a longitudinal centerline is shown. As noted previously, the absorbent system 40 may comprise a first absorbent core 460 and a second absorbent core 470. As shown, the first absorbent core 460 has an upper surface 460A and a lower surface 460B which opposes the upper surface. Similarly, the second absorbent core 470 has an upper surface 470A and a lower surface 470B. Additionally, the first absorbent core 460 and/or the second absorbent core 470 may comprise a laminate structure which includes a plurality of layers. Such forms are discussed in additional detail hereafter.

As shown, the first absorbent core 460 may be joined to the second absorbent core 470 in an offset manner or configuration along the length of the absorbent system 40. As used herein "offset" or "offset manner" means that the layers of interest are staggered and that their respective end edges are not aligned in a z-direction (i.e., the end edge of one layer or laminate structure is not coterminous with the end edge of an adjacent underlying or overlying layer or laminate structure) when the layers or laminate structures overlay one another. This offset joinder of the first and second absorbent core 460 and 470 results in an overlapping and joined area of the two layers that forms a central portion 205C of the absorbent system 40. The central portion 205C of the absorbent system 40 is consequently bounded on each side by a front end portion 205F and a rear end portion 205R, both of the absorbent system 40. In other words, the front end portion 205F and the rear end 205R portion are respectively disposed at opposing ends of the absorbent system 40. As shown, a distance between the end edge 66 and the end edge 76 can define a length of the front end portion 205F. Similarly, a distance between the end edge 477 and the end edge 467 can define a length of the rear end portion 205R. The end edge 466 may be the leading edge (more proximal to the first end 90 of the pad 10) of the absorbent system 40 while the end edge 477 may be the trailing edge (more proximal to the second end 92 of the pad 10) of the absorbent system 10.

The length of the central portion 205C can vary by size of the absorbent article 10. For example, for those absorbent articles sized for higher BMI wearers, the length of the central portion 205C can be higher than the central portion 205C for absorbent articles sized for wearers having a lower BMI. Additionally, where the absorbent articles are equipped with elasticated barrier leg cuffs, the central portion 205C may extend past the outermost anchor points of the elastomeric members of the barrier leg cuffs. Extension of the central portion 205C past these outermost anchor points can reduce the likelihood of the ends of the absorbent article folding during application of the absorbent article. Folding ends during application of the absorbent article can be problematic as described in U.S. Patent Application No. 2017/0049634. In some forms, the central portion 205C may have a length of at least 50mm, at least 75 mm, at least 90 mm, at least 100 mm, at least 125 mm, at least 150 mm, at least 175 mm, at least 200 mm, at least 225 mm, at least 250 mm, 275 mm, 300 mm, 325 mm, 350 mm, or 375 mm, specifically including all values within these ranges and any ranges created thereby.

It is worth noting that where the first absorbent core 460 and the second absorbent core 470 are positioned in an offset manner and are adhesively attached, care should be taken as to how the adhesive is applied. adhesive applied to the lower surface 460B should be strategically positioned to reduce the likelihood of contamination of the equipment. For example, as shown, adhesive applied in the front end portion 205F could contaminate the equipment as the second absorbent core 470 does not overly the adhesive in that area. Adhesive is needed in the central portion 205C. Additionally, adhesive should be provided in the rear end portion 205R. In such forms, adhesive would be applied to the carrier web to ensure that the second absorbent core 470 releases completely from a cut-and-slip or cut-and-lay operation. Where the second leading edge 476 forms the front end portion 205F, adhesive should be applied to the carrier web in the front end portion 205F and the central portion 205C to ensure that the second leading edge 476 is released from the cut-and-slip or cut-and-lay operation. Cut-and-slip and cut-and-lay devices are well known in the art.

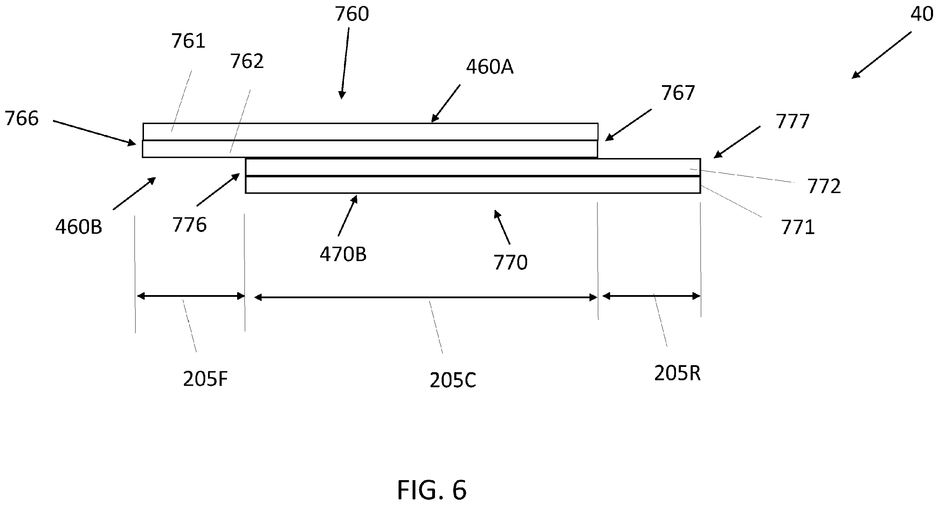

Referring now to FIG. 6, a first absorbent core 760 and/or a second absorbent core 770 may comprise a plurality of webs and layers themselves. For example, the first absorbent core 760 may comprise a first superabsorbent layer 761 disposed on a first distribution layer 762, i.e. a first absorbent core laminate 760. And, the second absorbent core 770 may comprise a second superabsorbent layer 771 disposed on a second distribution layer 772, i.e. a second absorbent core laminate 770. In some forms, the first distribution layer 762 is joined to the second distribution layer 772 in an offset manner or configuration along the length of the core. This offset joinder of the first and second distribution layers 762, 772 results in an overlapping and joined area of the two laminates that forms the central portion 205C of the absorbent system 40. As shown, the front end portion 205F is formed from the end edge 766 of the first absorbent core laminate 760 and the end edge 776 of the second absorbent core laminate 770. The rear end portion 205R of the absorbent system 40 is formed by the end edge 777 of the second absorbent core laminate 770 and the end edge 767 of the first absorbent core laminate 760.

It is worth noting that configurations are contemplated where the first distribution layer 762 is joined to the second super absorbent layer 771 rather than the second distribution layer 772. **In** such forms, the laminates may be joined to one another in an offset manner as well except the first distribution layer 762 is joined to the second superabsorbent layer 771 instead of the second distribution layer 772. Additionally, configurations are contemplated where at least one of the layers of the absorbent system 40 comprises a single layer, e.g. a superabsorbent layer or a distribution layer, while the other comprises a laminate structure as described herein.

As shown, the end edge 766 and end edge 777 of the first and second absorbent core laminates oppose each other and form the front end portion 205F and the rear end portion 205R of the absorbent system 205, respectively or vice versa. In other forms, the end edge 767 and end edge 777 of the first and second absorbent core laminates may oppose each other and form a front end portion 205F and a rear end portion 205R of the absorbent system 205, respectively or vice versa. In both instances, the end edge 766 and end edge 777 may be in the form of a male connection derived from a nested cut of the first and second absorbent cores. Similarly, the end edge 767 and end edge 776 may be in the form of a female connection derived from a nested cut of the first and second laminates, respectively.

In some forms, the overlapping area or region that forms the central portion 205C of the absorbent system 40 has at least one characteristic of a greater capacity, a greater void volume, or a greater thickness than the front end portion 205F and the rear end portion 205F of the absorbent system 205. These forms may be particularly useful for providing for heightened leakage protection in the central portion where female users of such pads would typically contact the pad along with increased flexibility in the front end portion and rear end portion.

The configuration and construction of the absorbent system 40 may vary (e.g., the absorbent system 40 may have varying caliper zones, a hydrophilic gradient, a superabsorbent gradient, or lower average density and lower average basis weight acquisition zones). Further, the size and absorbent capacity of the absorbent system 40 may also be varied to accommodate a variety of wearers. However, the total absorbent capacity of the absorbent system 40 should be compatible with the design loading and the intended use of the disposable absorbent article 10.

The absorbent system 40 may comprise a plurality of multi-functional layers that are in addition to the first and second absorbent cores. For example, the absorbent system 40 may comprise a core wrap (not shown) useful for enveloping the first and second laminates and other optional layers. The core wrap may be formed by two nonwoven materials, substrates, laminates, films, or other materials. In a form, the core wrap may only comprise a single material, substrate, laminate, or other material wrapped at least partially around itself.

The absorbent system 40 of the present disclosure may comprise one or more adhesives, for example, to help immobilize the SAP or other absorbent materials within the first and second laminates.

Absorbent cores comprising relatively high amounts of SAP with various core designs are disclosed in U.S. Pat. No. 5,599,335 to Goldman et al., EP 1,447,066; WO 95/11652; U.S. Pat. Publ. No. 2008/0312622A1; and WO 2012/052172. These may be used to configure the superabsorbent layers.

Additions to the core of the present disclosure are envisioned. In particular, potential additions to the current multi-laminate absorbent core are described in U.S. Pat. Nos. 4,610,678; 4,673,402; 4,888,231; 4,834,735; 5,234,423; and 5,147,345. These are useful to the extent they do not negate or conflict with the effects of the below described layers of the absorbent core of the present invention.

The first and second absorbent cores layers and/or laminates of the absorbent system 40 have been detailed earlier but it is important to note that these layers or laminates may have cross-direction widths that are the same as each other or different. As discussed previously, for example, the first absorbent core layer or laminate may have a lesser cross-direction width than said second absorbent core layer or laminate or a greater cross-direction width than said second absorbent core layer or laminate. In certain instances, the first and second absorbent core layers or laminates can have machine-direction lengths that are the same while in other instances, the first and second absorbent cores have machine-direction lengths that are different. In the latter instance, the first absorbent core layer or laminate may have a lesser machine-direction length than the second absorbent core layer or laminate, or conversely the first absorbent core layer or laminate may have a greater machine-direction length than said second absorbent core layer or laminate.

The first and second absorbent core layers or laminates may further comprise an optional intermediate layer disposed between the respective superabsorbent layer and distribution layer. This optional intermediate layer may comprise materials detailed herein relative to the optional layers for the chassis, in general.

As stated previously, in some forms, the first absorbent core layer or laminate has end edge 66 that is complementary in shape to its respective end edge 68. More specifically, the end edge 66 of the first absorbent core layer or laminate may conform shape wise to the end edge 68 of the same. The same conformance may apply to the second absorbent core layer or laminate. This conformation results from a nested cut of the first absorbent core layer or laminate and the second absorbent core layer or laminate that provides matching or shape fitting ends. Likewise, this feature may also be prevalent in any optional absorbent cores that might be incorporated into the absorbent system. This nesting or nested cut feature of the absorbent cores allow for reduced waste of trim during manufacture. It has also been found that it is possible to configure the first and second absorbent core layers or laminates in a manner that allows for their respective convex edges to oppose one another when the first and second layers are overlapped and joined forming an absorbent system with a central portion 205C comprising an overlapping area.

Referring to FIGS. 4 to 6, as noted previously, the front end portion of the absorbent system 205F can be formed from end edge 466 or end edge 477 of either the first absorbent core or the second absorbent core. A rear end portion of the absorbent system 205R may similarly be formed from end edge 466 or end edge 477 of the other of the first absorbent core or the second absorbent core. This configuration yields an absorbent system with matching (i.e., a male connection) ends. In other forms, a front end portion of the absorbent system may be formed from end edge 466 or end edge 477 of either the first absorbent core or the second absorbent core while the rear end portion of the absorbent system is formed from end edge 467 or end edge 476 of the other of the first absorbent core or second absorbent core. In such forms, the second end is shaped as a female connection and therefore does not match the front end portion of the same core. In other forms, the front end portion of the absorbent system may be formed from the end edge 467 of the first absorbent core or end edge 477 of the second absorbent core. A rear end portion of the absorbent system may be similarly formed from the end edge 467 of the remaining first absorbent core or the end edge 477 of the second absorbent core. This configuration yields an absorbent system with matching (i.e., a female connection) ends. It should be noted, however, that the width of the first and second absorbent cores may be the same or different as mentioned herein. The nested cuts of the end edges of each of the first and second absorbent cores can have shapes selected from the group consisting of arcs, semicircles, semi-ellipses, chevrons, rectangles, sinusoids, jigsaws, and combinations thereof.

In some forms, the first or second absorbent cores may include one or more recessed areas that run along the machine direction or cross direction. These recessed areas may coincide with the discontinuous patterns of one or more of a superabsorbent layer and distribution layer, whether it be of the first absorbent core, second absorbent core, or both. These recessed areas may also merely be formed by embossing of the first or second absorbent cores. These recessed areas may alternatively be formed by slitting, cutting, ring-rolling, or otherwise providing mechanical deformation through the first and/or second absorbent cores. Each manner of recessed area formation mentioned herein is intended to yield a recessed area that is capable of providing a point of preferential bending of the overall article.

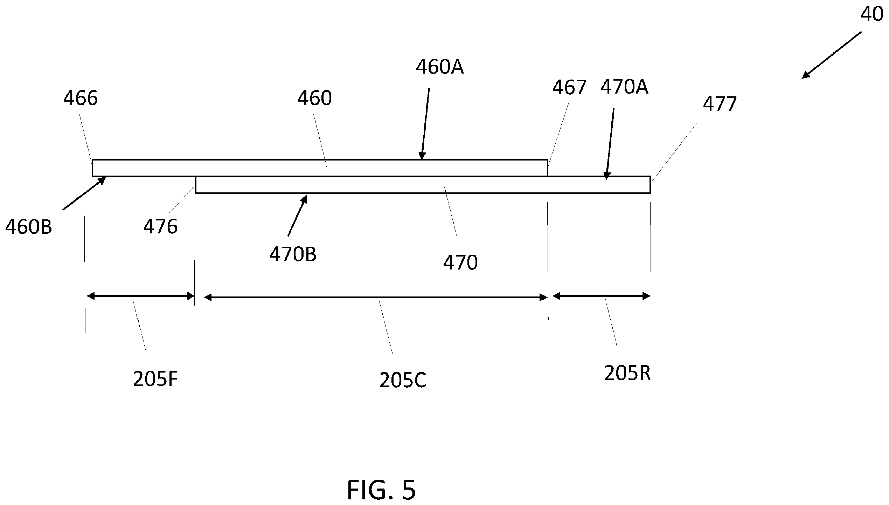

Additionally, for those forms where the first absorbent core and/or the second absorbent core do not comprise laminate structures, an airlaid core material can be utilized. Any suitable airlaid core can be utilized. Airlaid core material can be obtained by a manufacturer of such materials or can be made online via equipment known in the art. Where an airlaid core is utilized, the need for separate superabsorbent layers and distribution layers may be reduced. In such forms, the absorbent core web 500 (shown in FIG. 5) may comprise an airlaid web as described herein. Suitable airlaid absorbent core structures are disclosed in U.S. Patent Nos. 8,105,301 and 8,603,622 and U.S. Patent Application No. 2017/0348166.

Superabsorbent layers

Referring to FIGS. 4 and 6, the first and second superabsorbent layers 761, 771 of the first and second absorbent core laminates 760, 770 comprise superabsorbent polymers or absorbent gelling materials (AGM). In some forms, the superabsorbent layer 761 and/or 771 may comprise the carrier web and composition. In such forms, superabsorbent may be deposited on the carrier web to form the superabsorbent layers. The superabsorbent layers may comprise AGM particles or AGM fibers. In general, such AGM's have been used only for their fluid-absorbing properties. Such materials form hydrogels on contact with liquid (e.g., with urine, blood, and the like). One highly preferred type of hydrogel-forming, absorbent gelling material is based on the hydrolyzed polyacids, especially neutralized polyacrylic acid. Hydrogel-forming polymeric materials of this type are those which, upon contact with fluids (i.e., liquids) such as water or body fluids, imbibe such fluids and thereby form hydrogels. In this manner, fluid discharged into the fluid absorbent structures herein can be acquired and held. These preferred superabsorbent polymers will generally comprise substantially water-insoluble, slightly cross-linked, partially neutralized, hydrogel-forming polymer materials prepared from polymerizable, unsaturated, acid-containing monomers.

The size of the fluid absorbent gelling material particles may vary over a wide range. For reasons of industrial hygiene, average particle sizes smaller than about 30 microns are less desirable. Particles having a smallest dimension larger than about 2 mm may also cause a feeling of grittiness in the absorbent article, which is undesirable from a consumer aesthetics standpoint. Furthermore, rate of fluid absorption can be affected by particle size. Larger particles have very much reduced rates of absorption. Fluid absorbent gelling material particles preferably have a particle size of from about 30 microns to about 2 mm for substantially all of the particles. "Particle Size" as used herein means the weighted average of the smallest dimension of the individual particles.

In some forms, the absorbent cores or portions thereof of the present disclosure may be substantially free of airfelt and are thus distinct from mixed layers that may include airfelt. As used herein, "substantially free of airfelt" means less than 5%, 3%, 1%, or even 0.5% of airfelt. In some forms, there may be no measurable airfelt in the superabsorbent layers. In the case of the first superabsorbent layer, it is preferably disposed onto the first distribution layer discontinuously. And as noted previously, the second superabsorbent layer may, in conjunction with the first superabsorbent layer or independently thereof, be disposed on the second distribution layer discontinuously. As used herein "discontinuously" or "in a discontinuous pattern" means that the superabsorbent polymers are applied onto the first distribution layer in a pattern of disconnected shaped areas. These areas of superabsorbent polymers or areas free of superabsorbent polymer may include, but are not limited to linear strips, non-linear strips, circles, rectangles, triangles, waves, mesh, and combinations thereof. The first superabsorbent layer like the second superabsorbent layer may, however, be disposed onto its respective distribution layer in a continuous pattern. As used herein "continuous pattern" or "continuously" means that the material is deposited and or secured to a superabsorbent carrier material and/or the adjacent distribution layer in an uninterrupted manner such that there is rather full coverage of the distribution layer by the superabsorbent polymer.

In some forms, the first and second superabsorbent layers may comprise superabsorbent polymers that are the same. In other embodiments, the first and second superabsorbent layers may comprise superabsorbent polymers that are different from one another. This is may be in addition to the different deposition patterns that are discussed above.

The superabsorbent layers are disposed having a thickness of 0.2mm, 0.3mm, 0.4mm, or 0.5mm to 1mm, 1.2mm, 1.4mm, 1.8mm, or 2mm. The first and second superabsorbent layers may have the same or different cross-direction widths as applied to their respective distribution layers. For instance, the cross-direction widths of the first and second superabsorbent layers may be from 20mm, 25mm, 30mm, 35mm, or 40mm to 50mm, 60mm, 65mm, 70mm, 80mm, or 90mm. Alternatively, in embodiments where the widths of the first and second superabsorbent layers differ from one another in the cross-direction width, the first superabsorbent layer may have a lesser cross-direction width than the second superabsorbent layer. In particular, the first superabsorbent layer may have a cross-direction width that is less than about 95%, 90%, 80%, 70%, or even 60% of the width of the second superabsorbent layer.

In certain embodiments, the one or both of the first and second superabsorbent layers span greater than greater than about 50%, 60%, 70%, 80%, 90%, or even 95% of the cross-direction width of a superabsorbent carrier layer and/or the respective adjoining first or second distribution layer.

The super absorbent layers may further comprise a nonwoven carrier web. Carrier webs may be webs selected from the group consisting of a fibrous structure, an airlaid web, a wet laid web, a high loft nonwoven, a needle punched web, a hydroentangled web, a fiber tow, a woven web, a knitted web, a flocked web, a spunbond web, a layered spunbond/ melt blown web, a carded fiber web, a coform web of cellulose fiber and melt blown fibers, a coform web of staple fibers and melt blown fibers, and layered webs that are layered combinations thereof.

Carrier webs may comprise materials such as creped cellulose wadding, fluffed cellulose fibers, airfelt, and textile fibers. The materials of the webs may also be fibers such as, for example, synthetic fibers, thermoplastic particulates or fibers, tricomponent fibers, and bicomponent fibers such as, for example, sheath/core fibers having the following polymer combinations: polyethylene/polypropylene, polyethylvinyl acetate/polypropylene, polyethylene/polyester, polypropylene/polyester, copolyester/polyester, and the like. The optional layers may be any combination of the materials listed above and/or a plurality of the materials listed above, alone or in combination. The materials of the webs may be hydrophobic or hydrophilic depending on their placement within the chassis.

The materials of the webs may comprise constituent fibers comprising polymers such as polyethylene, polypropylene, polyester, and blends thereof. The fibers may be spunbound fibers. The fibers may be meltblown fibers. The fibers may comprise cellulose, rayon, cotton, or other natural materials or blends of polymer and natural materials. The fibers may also comprise a superabsorbent material such as polyacrylate or any combination of suitable materials. The fibers may be monocomponent, bicomponent, and/or biconstituent, non-round (e.g., capillary channel fibers), and may have major cross-sectional dimensions (e.g., diameter for round fibers) ranging from 0.1-500 microns. The constituent fibers of the nonwoven precursor web may also be a mixture of different fiber types, differing in such features as chemistry (e.g. polyethylene and polypropylene), components (mono- and bi-), denier (micro denier and >20 denier), shape (i.e., capillary and round) and the like. The constituent fibers may range from about 0.1 denier to about 100 denier.

The webs may include thermoplastic particulates or fibers. The materials, and in particular thermoplastic fibers, may be made from a variety of thermoplastic polymers including polyolefins such as polyethylene (e.g., PULPEX™) and polypropylene, polyesters, copolyesters, and copolymers of any of the foregoing.

Depending upon the desired characteristics, suitable thermoplastic materials include hydrophobic fibers that have been made hydrophilic, such as surfactant-treated or silica-treated thermoplastic fibers derived from, for example, polyolefins such as polyethylene or polypropylene, polyacrylics, polyamides, polystyrenes, and the like. The surface of the hydrophobic thermoplastic fiber may be rendered hydrophilic by treatment with a surfactant, such as a nonionic or anionic surfactant, e.g., by spraying the fiber with a surfactant, by dipping the fiber into a surfactant or by including the surfactant as part of the polymer melt in producing the thermoplastic fiber. Upon melting and resolidification, the surfactant will tend to remain at the surfaces of the thermoplastic fiber. Suitable surfactants include nonionic surfactants such as Brij 76 manufactured by ICI Americas, Inc. of Wilmington, Del., and various surfactants sold under the Pegosperse™ by Glyco Chemical, Inc. of Greenwich, Conn. Besides nonionic surfactants, anionic surfactants may also be used. These surfactants may be applied to the thermoplastic fibers at levels of, for example, from about 0.2 to about 1 $g/cm^2$ of thermoplastic fiber.

Suitable thermoplastic fibers may be made from a single polymer (monocomponent fibers) or may be made from more than one polymer (e.g., bicomponent fibers). The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.

Suitable bicomponent fibers for use in the webs of this disclosure may include sheath/core fibers having the following polymer combinations: polyethylene/polypropylene, polyethylvinyl acetate/polypropylene, polyethylene/polyester, polypropylene/polyester, copolyester/polyester, and the like. Particularly suitable bicomponent thermoplastic fibers for use herein are those having a polypropylene or polyester core, and a lower melting copolyester, polyethylvinyl acetate or polyethylene sheath (e.g., DANAKLON™, CELBOND™, or CHISSO™ bicomponent fibers). These bicomponent fibers may be concentric or eccentric. As used herein, the terms "concentric" and "eccentric" refer to whether the sheath has a thickness that is even, or uneven, through the cross-sectional area of the bicomponent fiber. Eccentric bicomponent fibers may be desirable in providing more compressive strength at lower fiber thicknesses. Suitable bicomponent fibers for use herein may be either uncrimped (i.e., unbent) or crimped (i.e., bent). Bicomponent fibers may be crimped by typical textile means such as, for example, a stuffer box method or the gear crimp method to achieve a predominantly two-dimensional or "flat" crimp.

The length of bicomponent fibers may vary depending upon the particular properties desired for the fibers and the web formation process. Typically, in an airlaid web, these thermoplastic fibers have a length from about 2mm to about 12mm long such as, for example, from about 2.5mm to about 7.5mm long, and from about 3.0mm to about 6.0mm long. Nonwoven fibers may be between 5 mm long and 75 mm long, such as, for example, 10 mm long, 15 mm long, 20 mm long, 25 mm long, 30 mm long, 35 mm long, 40 mm long, 45 mm long, 50 mm long, 55 mm long, 60 mm long, 65 mm long, or 70 mm long. The properties of these thermoplastic fibers may also be adjusted by varying the diameter (caliper) of the fibers. The diameter of these thermoplastic fibers is typically defined in terms of either denier (grams per 9000 meters) or decitex (grams per 10,000 meters). Suitable bicomponent thermoplastic fibers as used in an airlaid making machine may have a decitex in the range from about 1.0 to about 20 such as, for example, from about 1.4 to about 10, and from about 1.7 to about 7 decitex.

The compressive modulus of these thermoplastic materials, and especially that of the thermoplastic fibers, may also be important. The compressive modulus of thermoplastic fibers is affected not only by their length and diameter, but also by the composition and properties of the polymer or polymers from which they are made, the shape and configuration of the fibers (e.g., concentric or eccentric, crimped or uncrimped), and like factors. Differences in the compressive modulus of these thermoplastic fibers may be used to alter the properties, and especially the density characteristics, of the respective thermally bonded fibrous matrix.

The webs may also include synthetic fibers that typically do not function as binder fibers but alter the mechanical properties of the fibrous webs. Synthetic fibers include cellulose acetate, polyvinyl fluoride, polyvinylidene chloride, acrylics (such as Orlon), polyvinyl acetate, nonsoluble polyvinyl alcohol, polyethylene, polypropylene, polyamides (such as nylon), polyesters, bicomponent fibers, tricomponent fibers, mixtures thereof and the like. These might include, for example, polyester fibers such as polyethylene terephthalate (e.g., DACRON™, and KODEL™), high melting crimped polyester fibers (e.g., KODEL™ 431 made by Eastman Chemical Co.) hydrophilic nylon (HYDROFIL™), and the like. Suitable fibers may also hydrophilized hydrophobic fibers, such as surfactant-treated or silica-treated thermoplastic fibers derived from, for example, polyolefins such as polyethylene or polypropylene, polyacrylics, polyamides, polystyrenes, polyurethanes and the like. In the case of nonbonding thermoplastic fibers, their length may vary depending upon the particular properties desired for these fibers. Typically they have a length from about 0.3 to 7.5 cm, such as, for example from about 0.9 to about 1.5 cm. Suitable nonbonding thermoplastic fibers may have a decitex in the range of about 1.5 to about 35 decitex, such as, for example, from about 14 to about 20 decitex.

Distribution Layers

The first and second distribution layers are useful for wicking bodily fluids away from the skin of a wearer to facilitate comfort of continued wear after a release. In some forms, the support web may comprise the distribution layer. In some forms, the support web may be configured similar to the carrier web described herein. In some forms, the first and second distribution layers of the first and/or second laminates not only face one another but are joined in an offset manner to form part of the core. The distribution layers comprise one or more of cellulose and commuted wood pulp. This may be in the form of airlaid. The airlaid may be chemically or thermally bonded. In particular, the airlaid may be multi bonded airlaid (MBAL). In this instance, the distribution layer may further comprise a fibrous thermoplastic adhesive material at least partially bonding the airlaid to itself and adjacent distribution layers, superabsorbent layers, or other additional (optional) layers. It should be noted that the same materials that are suitable for the optional layers of the chassis are envisioned as suitable for use in the distribution layers. The basis weight for each of the first and second distribution layers range from 80gsm, 80 gsm, 100gsm, 110 gsm, 120gsm, or 130 gsm to 140 gsm, 150 gsm, 160 gsm, 180 gsm, 200 gsm, 220 gsm, or 240 gsm. A preferred basis weight is 135 gsm for each of the distribution layers of the first and second laminates. Forms are contemplated where the absorbent core web 500 (shown in FIG. 5) comprises a laminate structure of a superabsorbent layer and a distribution layer.

**Test Methods:**

Linear distances:

Linear distances may be measured by any appropriate instrument that is calibrated and capable of a measurement to the nearest 0.1 mm. Area measurements are made using the projected area of the article, as viewed orthogonally to the plane of the longitudinal and transverse axes, in square millimeters to the nearest 0.1 mm$^2$.

Article Length & Length of Visual Signal

The overall length of an absorbent article is measured as the distance between the front leading edge and rear leading edge along the longitudinal centerline of the absorbent article. The overall length of the visual signal that is visible on the wearer-facing side of the absorbent article is measured as the distance between the endpoint of the signal closest to the front leading edge of the article and the endpoint of the signal closest to the rear leading edge of the article, along an axis that is parallel to the longitudinal axis of the article. The length of the visual signal is taken as the maximum length that the printed signal covers in the longitudinal direction of the absorbent article, even if this maximum length does not occur directly at the longitudinal centerline. All testing is performed in a room maintained at a temperature of 23° C ± 2.0° C and a relative humidity of 50% ± 2% and samples are conditioned under the same environmental conditions for at least 2 hours prior to testing.

To prepare the test sample, first remove it from any wrapper present and remove the release paper, if necessary, to expose the Panty Fastening Adhesive (PFA) on the garment-facing side of the absorbent article. Apply a light dusting of talc powder to the PFA to mitigate tackiness. Suspend the article vertically by its front leading edge. Attach a 500 g ± 1 g weight to the rear leading edge allowing the article to hang freely. After 30 seconds, measure the length of the article along the longitudinal centerline using a calibrated steel metal ruler traceable to NIST, or equivalent. Record as Article Length to the nearest 0.1 mm.

Now mount the article on a flat metal plate that is approximately 3 mm thick with a length and width larger than the article. Using masking tape (about 2.54 cm wide), secure the article to the center of the metal plate as follows. With the garment-facing side of the article facing the metal plate, attach a strip of tape across the front leading edge of the article in such a way that no portion of the tape overlaps the printed visual signal. In like fashion, the rear leading edge of the article is secured to the metal plate such that the article is extended to the previously measured Article Length. Determine the endpoint of the printed visual signal that is closest to the front leading edge, then draw a straight, fine line at this endpoint that is perpendicular to the longitudinal axis of the article. In like fashion, draw another fine line at the rear leading edge that corresponds to the endpoint of the visual signal. Using a calibrated steel metal ruler traceable to NIST, or equivalent, measure the distance between the visual signal endpoint lines along an axis that is parallel to the longitudinal axis of the article. Record as Visual Signal Length to the nearest 0.1mm. The percentage of the Visual Signal Length versus the Article Length is calculated as follows

$$\% \text{ Visual Signal Length} = (\text{ Visual Signal Length} / \text{ Article Length }) * 100$$

and reported as % Visual Signal Length to the nearest 0.1 %.

In like fashion, a total of five replicate test samples are prepared and analyzed. The arithmetic mean is calculated for % Visual Signal Length and reported to the nearest 0.1%.

Caliper

The thickness of a test specimen is measured as the distance between a reference platform on which the specimen rests and a pressure foot that exerts a specified amount of pressure onto the specimen over a specified amount of time. All measurements are performed in a laboratory maintained at 23 °C ± 2 C° and 50% ± 2% relative humidity and test specimens are conditioned in this environment for at least 2 hours prior to testing.

Specimen thickness is measured with a manually-operated micrometer equipped with a pressure foot capable of exerting a steady pressure of 0.10 psi ± 0.01 psi onto the test specimen. The manually-operated micrometer is a dead-weight type instrument with readings accurate to 0.01 mm. A suitable instrument is Mitutoyo Series 543 ID-C Digimatic, available from VWR International, or equivalent. The pressure foot is a flat ground circular movable face with a diameter that is smaller than the test specimen and capable of exerting the required pressure. A suitable pressure foot has a diameter of 40 mm; however, a smaller or larger foot can be used depending on the size of the specimen being measured. The test specimen is supported by a horizontal flat reference platform that is larger than and parallel to the surface of the pressure foot. The system is calibrated and operated per the manufacturer's instructions.

Obtain a test specimen by removing it from an absorbent article, if necessary. When excising the test specimen from an absorbent article, use care to not impart any contamination or distortion to the test specimen layer during the process. The test specimen is obtained from an area free of folds or wrinkles, and it must be larger than the pressure foot.

To measure thickness, first zero the micrometer against the horizontal flat reference platform. Place the test specimen on the platform with the test location centered below the pressure foot. Gently lower the pressure foot with a descent rate of 3.0 mm ± 1.0 mm per second until the full pressure is exerted onto the test specimen. Wait 5 seconds and then record the thickness of the test specimen to the nearest 0.01 mm. In like fashion, repeat for a total of ten replicate test specimens. Calculate the arithmetic mean for all thickness measurements and report as Thickness to the nearest 0.01 mm.

The dimensions and values disclosed herein are not to be understood as being strictly limited to the exact numerical values recited. For example, a dimension disclosed as "40 mm" is intended to mean "about 40 mm."

**Claims**

1. A disposable absorbent article having a longitudinal centerline and a lateral centerline generally perpendicular to the longitudinal centerline, first and second side edges and first and second end edges disposed at a first end and a second end of the absorbent article, respectively, the absorbent article further comprising:

   a topsheet;
   a backsheet;
   an absorbent system disposed between the topsheet and the backsheet; and
   a visual signal visible from a wearer-facing surface of the disposable absorbent article, wherein a difference between a length of the disposable absorbent article and a length of the visual signal is less than 35 mm as determined by the Article Length & Length of Visual Signal method;
   wherein the absorbent article exhibits a performance factor of between 2 and 13;
   wherein the performance factor is the difference between the article length and visual signal length divided by the measured caliper as determined by the Article Length & Length of Visual Signal and Caliper methods as described herein .

2. The disposable absorbent article of claim 1, wherein at least one of the layers of the absorbent system is comprised by at least one of the first end edge or the second end edge.

3. The disposable absorbent article of any of the preceding claims, wherein the absorbent system comprises a secondary topsheet and an absorbent core.

4. The disposable absorbent article of claim 3, wherein the secondary topsheet is comprised by the first end edge and/or the second end edge

5. The disposable absorbent article of any of the preceding claims, wherein the visual signal has a length of between about 140 mm to about 230 mm, more preferably from about 150 mm to about 220 mm, or most preferably from about 160 mm to about 210 mm.

6. The disposable absorbent article of any of the preceding claims, wherein the absorbent article has an overall length of between 160 mm to about 260 mm, more preferably from about 170 mm to about 250 mm, or most preferably from about 180 mm to about 240 mm.

7. The disposable absorbent article of any of the preceding claims, wherein the visual signal length is at least about 84 percent of the absorbent article length, more preferably at least about 86 percent, or most preferably about 88 percent.

8. The disposable absorbent article of any of the preceding claims, further comprising a pair of longitudinal pleats, at least one of the pleats being disposed adjacent the first side edge and the other of the pair of longitudinal pleats being disposed adj acent the second side edge.

9. The disposable absorbent article of any of claims 1-4, wherein the visual signal has a length of at between about 220 mm to about 400 mm, more preferably from about 235 mm to about 390 mm, or most preferably from about 240 mm to about 370 mm.

10. The disposable absorbent article of any of claims 1-4 and 9, wherein the absorbent article has an overall article length of between 230 mm to about 430 mm, more preferably from about 240 mm to about 420 mm, or most preferably from about 250 mm to about 410 mm.

11. The disposable absorbent article of any of claims 1-4 and 9-10, wherein the visual signal is between 80 percent to about 98 percent of the length of the absorbent article, more preferably from about 85 percent to about 95 percent of the length of the absorbent article, most preferably from about 90 percent to about 93 percent of the length of the absorbent article.

12. The disposable absorbent article of any of claims 1-4 and 9-11, further comprising a pair of longitudinal cuffs, at least one of the cuffs being disposed adjacent the first side edge and the other of the pair of longitudinal cuffs being disposed adjacent the second side edge.

13. The disposable absorbent article of any of the preceding claims wherein the visual signal is printed on the absorbent system.

14. The disposable absorbent article of any of the preceding claims wherein the disposable absorbent article comprises an incontinence pad.


**Patentansprüche**

1. Einweg-Absorptionsartikel, der eine Längsachse und eine Querachse, die im Allgemeinen lotrecht zu der Längsachse steht, erste und zweite Seitenränder und erste und zweite Endränder aufweist, die an einem ersten Ende beziehungsweise einem zweiten Ende des Absorptionsartikels angeordnet sind, der Absorptionsartikel umfassend:

   eine Oberschicht;
   eine Unterschicht;
   ein Absorptionssystem, das zwischen der Oberschicht und der Unterschicht angeordnet ist; und
   ein visuelles Signal, das von einer trägerseitigen Oberfläche des Einweg-Absorptionsartikels aus sichtbar ist, wobei ein Unterschied zwischen einer Länge des Einweg-Absorptionsartikels und einer Länge des visuellen Signals weniger als 35 mm beträgt, wie bestimmt durch das Verfahren der Artikellänge & Länge des visuellen Signals;
   wobei der Absorptionsartikel einen Leistungsfaktor zwischen 2 und 13 vorweist;
   wobei der Leistungsfaktor die Differenz zwischen der Artikellänge und der visuellen Signallänge geteilt durch die gemessene Stärke ist, wie bestimmt durch die hierin beschriebenen Verfahren der Artikellänge & Länge des visuellen Signals und der Stärke.

2. Einweg-Absorptionsartikel nach Anspruch 1, wobei mindestens eine der Schichten des Absorptionssystems durch mindestens einen des ersten Endrands oder des zweiten Endrands umfasst ist.

3. Einweg-Absorptionsartikel nach einem der vorstehenden Ansprüche, wobei das Absorptionssystem eine sekundäre Oberschicht und einen Absorptionskern umfasst.

4. Einweg-Absorptionsartikel nach Anspruch 3, wobei die sekundäre Oberschicht durch den ersten Endrand oder den zweiten Endrand umfasst ist

5. Einweg-Absorptionsartikel nach einem vorstehenden Anspruch, wobei das visuelle Signal eine Länge zwischen etwa 140 mm bis etwa 230 mm, mehr bevorzugt von etwa 150 mm bis etwa 220 mm, oder am meisten bevorzugt von etwa 160 mm bis etwa 210 mm aufweist.

6. Einweg-Absorptionsartikel nach einem der vorstehenden Ansprüche, wobei der Absorptionsartikel eine Gesamtlänge zwischen 160 mm bis etwa 260 mm, mehr bevorzugt von etwa 170 mm bis etwa 250 mm oder am meisten bevorzugt von etwa 180 mm bis etwa 240 mm aufweist ausweist.

7. Einweg-Absorptionsartikel nach einem der vorstehenden Ansprüche, wobei die Länge des visuellen Signals mindestens etwa 84 Prozent der Länge des Absorptionsartikels beträgt, mehr bevorzugt mindestens etwa 86 Prozent oder am meisten bevorzugt etwa 88 Prozent.

**8.** Einweg-Absorptionsartikel nach einem der vorstehenden Ansprüche, ferner umfassend ein Paar von Längsfältelungen, wobei mindestens eine der Fältelungen benachbart an den ersten Seitenrand angeordnet ist und die andere des Paars von Längsfältelungen benachbart an den zweiten Seitenrand angeordnet ist.

**9.** Einweg-Absorptionsartikel nach einem der Ansprüche 1 bis 4, wobei das visuelle Signal eine Länge zwischen etwa 220 mm bis etwa 400 mm, mehr bevorzugt von etwa 235 mm bis etwa 390 mm, oder am meisten bevorzugt von etwa 240 mm bis etwa 370 mm aufweist.

**10.** Einweg-Absorptionsartikel nach einem der Ansprüche 1 bis 4 und 9, wobei der Absorptionsartikel eine Gesamtartikellänge zwischen 230 mm bis etwa 430 mm, mehr bevorzugt von etwa 240 mm bis etwa 420 mm oder am meisten bevorzugt von etwa 250 mm bis etwa 410 mm aufweist.

**11.** Einweg-Absorptionsartikel nach einem der Ansprüche 1 bis 4 und 9 bis 10, wobei das visuelle Signal zwischen 80 Prozent bis etwa 98 Prozent der Länge des Absorptionsartikels beträgt, mehr bevorzugt von etwa 85 Prozent bis etwa 95 Prozent der Länge des Absorptionsartikels, am meisten bevorzugt von etwa 90 Prozent und etwa 93 Prozent der Länge des Absorptionsartikels.

**12.** Einweg-Absorptionsartikel nach einem der Ansprüche 1 bis 4 und 9 bis 11, ferner umfassend ein Paar von Längsbündchen, wobei mindestens eines der Bündchen benachbart an den ersten Seitenrand angeordnet ist und das andere des Paars von Längsbündchen benachbart an den zweiten Seitenrand angeordnet ist.

**13.** Einweg-Absorptionsartikel nach einem der vorstehenden Ansprüche, wobei das visuelle Signal auf das Absorptionssystem aufgedruckt ist.

**14.** Einweg-Absorptionsartikel nach einem der vorstehenden Ansprüche, wobei der Absorptionsartikel eine Inkontinenzeinlage umfasst.

**Revendications**

**1.** Article absorbant jetable ayant une ligne médiane longitudinale et une ligne médiane latérale généralement perpendiculaire à la ligne médiane longitudinale, des premier et second bords latéraux et des premier et second bords extrêmes disposés au niveau d'une première extrémité et d'une seconde extrémité de l'article absorbant, respectivement, l'article absorbant comprenant en outre :

une feuille de dessus ;
une feuille de fond ;
un système absorbant disposé entre la feuille de dessus et la feuille de fond ; et
un signal visuel visible à partir d'une surface faisant face au porteur de l'article absorbant jetable, dans lequel une différence entre une longueur de l'article absorbant jetable et une longueur du signal visuel est inférieure à 35 mm telle que déterminée par le procédé de longueur d'article et de longueur de signal visuel ;
dans lequel l'article absorbant présente un facteur de performance compris entre 2 et 13 ;
dans lequel le facteur de performance est la différence entre la longueur d'article et la longueur de signal visuel divisée par l'épaisseur mesurée telle que déterminée par les procédés de longueur d'article et de longueur de signal visuel et d'épaisseur tels que décrits ici.

**2.** Article absorbant jetable selon la revendication 1, dans lequel au moins l'une des couches du système absorbant est comprise par au moins l'un du premier bord extrême ou du second bord extrême.

**3.** Article absorbant jetable selon l'une quelconque des revendications précédentes, dans lequel le système absorbant comprend une feuille de dessus secondaire et une âme absorbante.

**4.** Article absorbant jetable selon la revendication 3, dans lequel la feuille de dessus secondaire est comprise par le premier bord extrême et/ou le second bord extrême

**5.** Article absorbant jetable selon l'une quelconque des revendications précédentes, dans lequel le signal visuel a une longueur comprise d'environ 140 mm à environ 230 mm, plus préférablement d'environ 150 mm à environ 220 mm, ou le plus préférablement d'environ 160 mm à environ 210 mm.

**6.** Article absorbant jetable selon l'une quelconque des revendications précédentes, dans lequel l'article absorbant a une longueur globale comprise de 160 mm à environ 260 mm, plus préférablement d'environ 170 mm à environ 250 mm, ou le plus préférablement d'environ 180 mm à environ 240 mm.

**7.** Article absorbant jetable selon l'une quelconque des revendications précédentes, dans lequel la longueur de signal visuel vaut au moins environ 84 pour cent de la longueur d'article absorbant, plus préférablement au moins environ 86 pour cent, ou le plus préférablement environ 88 pour cent.

**8.** Article absorbant jetable selon l'une quelconque des revendications précédentes, comprenant en outre une paire de plissages longitudinaux, au moins l'un des plissages étant disposé adjacent au premier bord latéral et l'autre parmi la paire de plissages longitudinaux étant disposé adjacent au second bord latéral.

**9.** Article absorbant jetable selon l'une quelconque des revendications 1 à 4, dans lequel le signal visuel a une longueur comprise d'environ 220 mm à environ 400 mm, plus préférablement d'environ 235 mm à environ 390 mm, ou le plus préférablement d'environ 240 mm à environ 370 mm.

**10.** Article absorbant jetable selon l'une quelconque des revendications 1 à 4 et 9, dans lequel l'article absorbant a une longueur globale d'article comprise de 230 mm à environ 430 mm, plus préférablement d'environ 240 mm à environ 420 mm, ou le plus préférablement d'environ 250 mm à environ 410 mm.

**11.** Article absorbant jetable selon l'une quelconque des revendications 1 à 4 et 9 à 10, dans lequel le signal visuel représente de 80 pour cent à environ 98 pour cent de la longueur de l'article absorbant, plus préférablement d'environ 85 pour cent à environ 95 pour cent de la longueur de l'article absorbant, le plus préférablement d'environ 90 pour cent à environ 93 pour cent de la longueur de l'article absorbant.

**12.** Article absorbant jetable selon l'une quelconque des revendications 1 à 4 et 9 à 11, comprenant en outre une paire de revers longitudinaux, au moins l'un des revers étant disposé adjacent au premier bord latéral et l'autre parmi la paire de revers longitudinaux étant disposé adjacent au second bord latéral

**13.** Article absorbant jetable selon l'une quelconque des revendications précédentes dans lequel le signal visuel est imprimé sur le système absorbant.

**14.** Article absorbant jetable selon l'une quelconque des revendications précédentes dans lequel l'article absorbant jetable comprend une serviette d'incontinence.

FIG. 1A

EP 3 975 964 B1

EP 3 975 964 B1

20

102    42    40    30    104

50    44

FIG. 1B

FIG. 1C

FIG. 2

FIG. 3

379

477

470

40

467

371

373

363

361

460

476

466

FIG. 4

369

FIG. 5

FIG. 6

EP 3 975 964 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008249494 A1
- US 2014343525 A1
- US 2019117475 A1
- US 2016051419 A1
- WO 9925295 A
- WO 9707764 A
- US 62772750 A
- US 20170049634 A1
- US 3929135 A
- US 4324246 A
- US 4342314 A
- US 4463045 A
- US 5006394 A
- US 4609518 A
- US 4629643 A
- US 4950264 A
- US 4988344 A
- US 4988345 A
- US 3978185 A
- US 7785690 B
- US 7838099 B
- US 5792404 A
- US 5665452 A
- US 8728049 B
- US 7553532 B
- US 7172801 B
- US 8440286 B
- US 7648752 B
- US 7410683 B
- US 7655176 B
- US 7402723 B
- US 8614365 B
- US 8704036 B
- US 6025535 A
- US 13743M
- US 20170258647 A1
- US 20160167334 A1
- US 20160166443 A1
- US 20170258651 A1
- US 5628097 A, Benson

- US 5885265 A
- GB 2184389 A
- GB 2184390 A
- GB 2184391 A
- US 4591523 A
- US 3989867 A
- US 3156242 A
- WO 9724097 A
- US 6623464 B
- US 6664439 B
- US 6436508 B
- US 6462251 B
- US 3881489 A
- US 4341216 A
- US 4713068 A
- US 4818600 A
- EP 203821 A
- EP 710471 A
- EP 710472 A
- EP 793952 A
- US 20150351976 A1
- US 20140343523 A1
- US 20180098893 A1
- US 62826178 A
- US 20170049634 A
- US 5599335 A, Goldman
- EP 1447066 A
- WO 9511652 A
- US 20080312622 A1
- WO 2012052172 A
- US 4610678 A
- US 4673402 A
- US 4888231 A
- US 4834735 A
- US 5234423 A
- US 5147345 A
- US 8105301 B
- US 8603622 B
- US 20170348166 A